# EUROPEAN PATENT SPECIFICATION

(11) **EP 2 361 066 B1**
(45) Date of publication and mention of the grant of the patent: **26.08.2015**
(21) Application number: 09753164.4
(22) Date of filing: 15.10.2009
(51) Int. Cl.: A61F 6/22

(54) **OCCLUSIVE PLUG**
VERSCHLUSSPFROPF
BOUCHON OCCLUSIF

(30) Priority: 15.10.2008 GB 0818852
(43) Date of publication of application: 31.08.2011
(73) Proprietor: O.I. Medical Limited, Sheffield South Yorkshire S10 5BY (GB)
(72) Inventor: Everingham John Sanders, Fulwood, Sheffield South Yorkshire S10 3TE (GB); Filshie Gilbert Marcus, Nottingham, Nottinghamshire NG3 5BG (GB); Sweeney Bernard, Thames Ditton, Surrey KT7 0TJ (GB); Magos Adam Laszlo, Barnet, Hertfordshire EN5 4EE (GB)
(74) Representative: Franks & Co Limited
(86) International application number: PCT/GB2009/002450
(87) International publication number: WO 2010/043857

(56) References cited:
- EP-A1- 1 800 633
- US-A1- 2004 204 720
- US-A1- 2005 217 680

## Description

### Field of the Invention

This invention relates to an occlusive plug for use in occluding a fallopian tube hysteroscopically and hence effective as a means of sterilisation.

### Background of the Invention

The principle of a threaded plug to be retained within the fallopian tube is known from WO 97/12569 which describes a plug in the form of an elongate body having an external thread, which plug, upon rotation, screws itself into the fallopian tube, the plug being self- retaining, and effective when in situ for purposes of sterilisation. Clearly the plug dimensions need to be selected according to the species to be sterilised, with the dual requirement of security against migration which might be caused by muscular tubal peristalsis, yet an ability to remove the plug at a later date should it be required to reverse the sterilisation procedure. In WO 2007/072004 is described an improved plug less prone to ejection and hence sterilisation failure, by providing a thread with a large root length and a large pitch. The proposal was to produce the plug in implantable grade polyethylene and various dimensions and ratios were advanced for thread forms to meet the practical requirements. Object of the invention.

A basic object of the present invention is the provision of a further improved plug.

### Summary of the Invention

According to a first aspect of the present invention there is provided a plug according to claim 1.

### Advantages of the Invention

After insertion of the plug, tissue within the lumen will bind onto the plug to provide enhanced plug location, and occlude the lumen, whereby the roughing and/or pitting reduces the possibility of post operative migration of the plug resulting from muscular tubal peristalsis.

The thread are. fenestrated. The fenestration enables endosalpinx and muscular tissue growth through the holes in the thread to aid anchorage of the plug occluding the lumen. This would also reduce the possibility of post operative migration of the plug. Such tissue growth would of course make the plug more difficult to remove should reversal of sterilisation be required, at some future date, but it is believed that the lumen would quickly regenerate.

Preferably, the leading end of the elongate body is provided with an extension nose having a smooth external surface and a rounded terminal end.

The extension nose constitutes a pilot nose providing self centring capability as the plug is inserted into the lumen. As the lumen is often tortuous this feature is most advantageous in reducing the risk of the plug creating a false passage, and hence being less effective in its role of sterilisation. Preferably, the leading end of the elongate body is provided with a conic helix of the same hand as the external thread, generally forming a continuation of the external thread. The pilot nose in the form of a conic helix constitutes a lumen finder in assisting initial location in, and penetration of the lumen. Preferably there is provided with a rotary drive tool, for inserting the plug hysteroscopically into a fallopian tube, the tool incorporating an integral flexible hysteroscope and comprising an outer sleeve housing a flexible drive rod adapted, at one end, to engage and apply torque to, the drive head of the plug with the other end of the drive rod being manually rotatable by the surgeon.

### Preferred or Optional Features

The plug is injection moulded in a rigid, inert, bio-compatible grade synthetic plastics.

The synthetic plastics is polyethylene.

The body and/or external thread is/are roughened and/or pitted during injection moulding.

The body and/or external thread is/are roughened and/or pitted by laser subsequently to moulding.

Roughening and/or pitting is up to 1 mm depth.

Fenestration is achieved by providing fine holes, approximately 1 mm diameter or less through the threads.

The extension nose is co-axial with the elongate body and is of circa 10mm length. The plug is of length 1.25cm to 3.5cm.

The plug is of diameter 2mm to 5mm.

The extension/pilot nose of the plug is engaged with the ostia of the fallopian tube/lumen to be occluded, by temporary attachment of the plug to one end of a drive tool, the latter incorporating an integral flexible hysteroscope and comprising an outer sleeve housing a flexible drive rod adapted, at one end to engage, and apply torque to, the trailing end of the elongate body of the plug, with the other end of the drive rod being manually rotatable by the surgeon.

### Brief Description of the Drawings

The invention will now be described in greater detail, by way of examples with reference to the accompanying drawings, in which:-
Figure 1 is a perspective view to an enlarged scale, of a non-claimed plug;
Figure 2 is an enlargement of a portion of Figure 1;
Figure 3 shows the plug of Figures 1 and 2 after insertion.
Figure 4 is a perspective view to an enlarged scale, of a plug according to the invention.
Figure 5 is an enlargement of a portion of Figure 4;
Figure 6 is a side elevation of the plug of figures 4 and 5;
Figure 7 is a perspective view to an enlarged scale, of a non-claimed plug;
Figure 8 shows the plug of Figure 6, after insertion;
Figure 9 is a perspective view to an enlarged scale, of a non-claimed plug;
Figure 10 shows a portion of Figure 8; and
Figure 11 shows a helix pilot portion.

In all embodiments, like parts are accorded like reference numerals. A plug 1 for use in occluding a fallopian tube hysteroscopically comprises an elongate body 2 having an external thread 3, preferably being 1.25cm to 3.5cm in length, and 2mm to 5mm in diameter.

The plug 1 is injection moulded in a rigid, inert, bio-compatible synthetic plastics grade polyethylene.

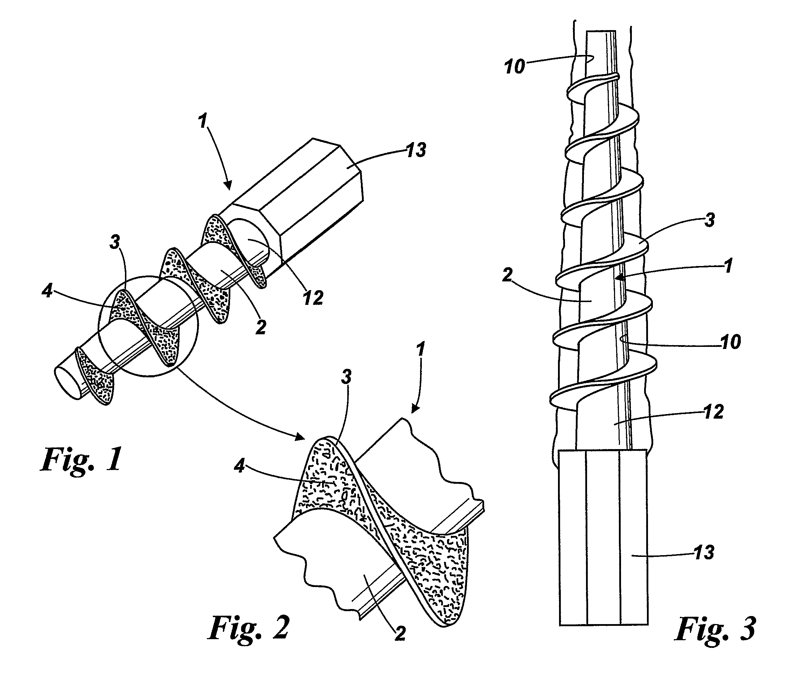

In accordance with the example illustrated in Figures 1 to 3, the body 2 and/or external thread 3 is roughened or pitted at 4.

The roughing or pitting is effected by a laser after injection moulding to a depth <1 mm.

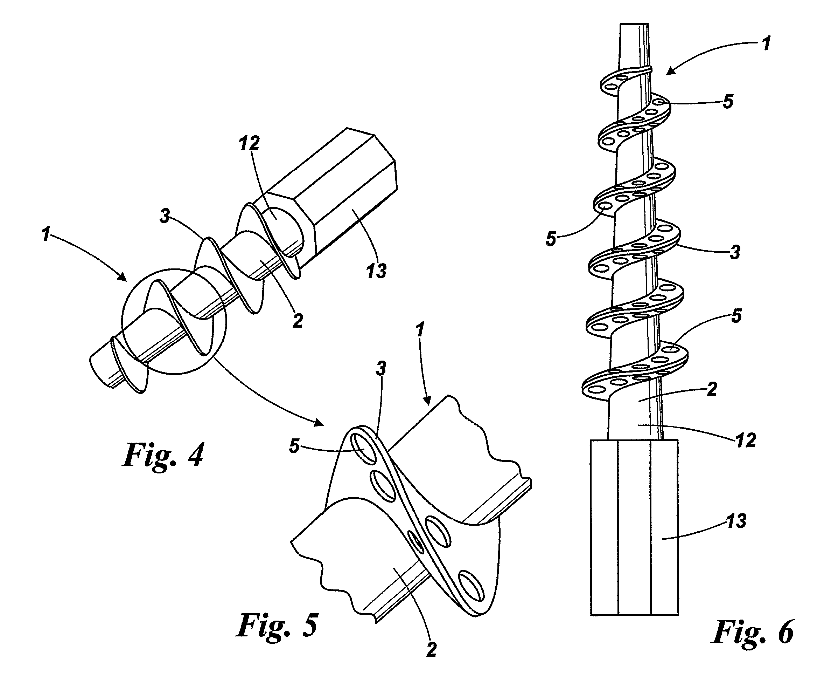

Of course, instead of pitting an injection moulded plug e.g. by laser, the roughening of an otherwise smooth surface could be achieved by pitting the surfaces of the mould tools e.g. by spark erosion, so that projections of polyethylene extend from the body 2 and/or thread 3 by a height of <1 mm. In the embodiment, illustrated in figures 4 to 6, pitting of the thread 3 is effective to such an extent that he thread is pierced or perforated by the formation of micropores 5 of e.g. 0.01 mm to 0.1 mm diameter.

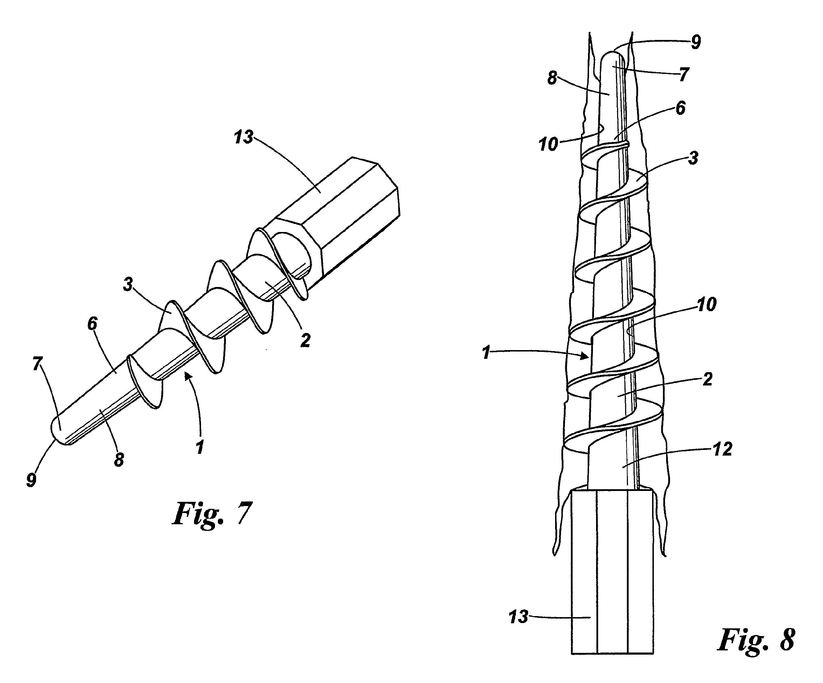

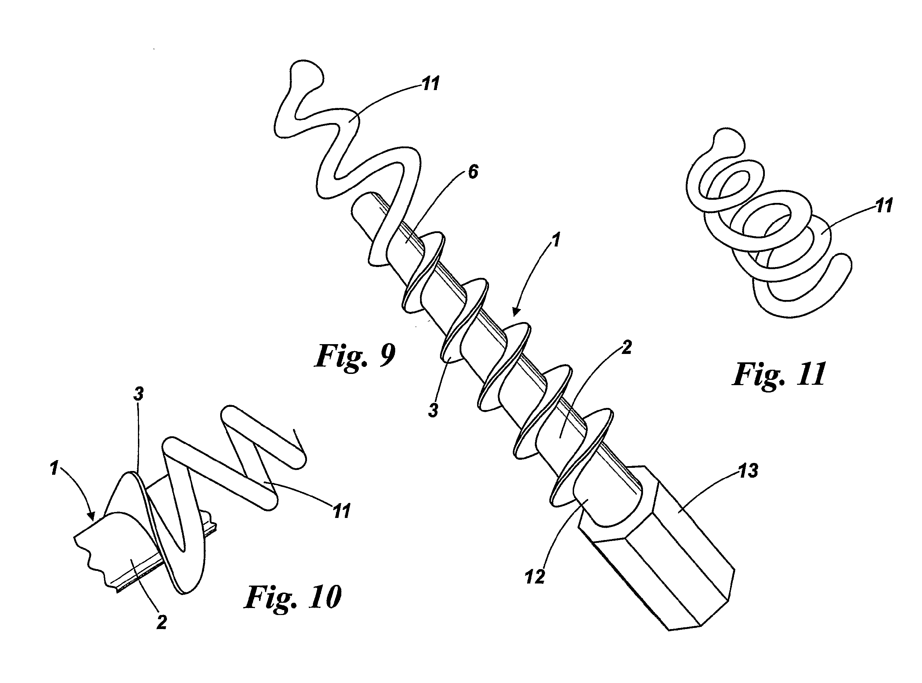

In the example illustrated in Figures 7 and 8, leading end 6 of the elongate body 2 is provided with an extension nose 7 having a smooth external surface 8 and a rounded terminal end 9 to assist penetration of the lumen 10. In the example illustrated in Figures 9 to 11, the leading end 6 is provided with a conic helix 11 of the same hand as the thread 3 and generally forms a continuation of the thread 3. At its trailing end 12, the plug 1 is provided a hexagonal drive head 13 for temporary attachment to a suitable connector of rotary drive tool (not shown) incorporating an integral flexible hysteroscope, the leading end 6 of the plug 1 being engaged with the ostia of a fallopian tube, to be occluded and the plug 1 then slowly rotated by the surgeon, to screw the plug 1 into and along the lumen 10, the drive tool basically comprising an outer sleeve housing a flexible drive rod adapted to be drivably engageable with, and readily releasably from, the drive head 13 of a plug 1, after completion of insertion.

## Claims

1. A plug (1) for use in occluding a fallopian tube hysteroscopically, said plug comprising:
an elongate body (2);
said elongate body having an external thread (3) for screwing the plug into and along a lumen,
said plug comprises a trailing end comprising a drive head (13), said drive head being adapted to be temporarily engaged by a portion of a drive tool in use, for rotation of said plug into and along a said lumen;
said drive head being adapted to be readily releasable from said drive tool after completion of insertion of the plug into said fallopian tube; and
**characterised in that**
said body (2) and/or said external thread (3) has/ have a roughened and/ or pitted external surface (4) for reducing post operative migration of said plug; and
said thread (3) is fenestrated.

2. A plug (1) as claimed in claim 1, wherein a leading end (6) of the elongate body (2) is provided with an extension nose (7) having a smooth external surface (8) and a rounded terminal end (9).

3. A plug as claimed in claim 1 or claim 2, which is injection moulded in a rigid, inert bio-compatible grade synthetic plastics material.

4. A plug as claimed in claim 3, wherein the plastics material is polyethylene.

5. A plug as claimed in any one of the preceding claims, wherein said roughening and/or said pitting is up to 1 mm in depth.

6. A plug as claimed in claim 2, wherein the extension nose (7) is coaxial with the elongate body (2) and is of circa 10mm length.

7. A plug as claimed in claim 1, comprising a fenestration consisting of a hole through said thread, said hole having a diameter of 1mm.

8. A plug as claimed in any preceding claim, of length 1.25 cm to 3.5 cm.

9. A plug as claimed in any preceding claim, of diameter 2 mm to 5 mm.

## Patentansprüche

1. Ein Stopfen (1) für Verwendung zum hysteroskopischen Verstopfen eines Eileiters, der Stopfen umfassend:
einen verlängerten Körper (2);
der verlängerte Körper habende ein Aussengewinde (3) für Schrauben des Stopfens in das Lumen und entlang dem Lumen,
der Stopfen umfassend ein Hinterende umfassend einen Antriebkopf (13), der Antriebkopf angepasst ist, zeitweilig mit einem Teil eines Antriebmittels im Verbrauch angestellt zu werden, für Drehung des Stopfens in das Lumen und entlang dem Lumen;
der Antriebkopf angepasst ist, bereit aus dem Antriebmittel nach Insertionabschluss des Stopfens in den Eileiter, entlassbar zu sein; und
**dadurch gekennzeichnet, dass**
der Körper (2) und/oder das Aussengewinde (3) eine aufgeraute und/oder vertiefte Aussenoberfläche (4) umfassen für Reduzierung postoperative Migration des Stopfens; und das Gewinde (3) gefenstert ist.

2. Ein Stopfen (1) nach Anspruch 1, wobei ein Vorderende (6) des verlängerten Körpers (2) mit einer Verlängerungsnase (7) mit einer glatten Aussenoberfläche (8) und einem Rundanschlussend (9) versehen ist.

3. Ein Stopfen nach Anspruch 1 oder Anspruch 2, der spritzgegossen in einem starren, inerten, biokompatibel Grad, synthetischen Kunstoff ist.

4. Ein Stopfen nach Anspruch 3, wobei der Kunststoff Polyethylen ist.

5. Ein Stofen nach einem der vorhergehenden Ansprüche, wobei das Aufrauen und/oder die Vertiefung bis zu 1 mm Tiefe ist.

6. Ein Stopfen nach Anspruch 2, wobei die Verlängerungsnase (7) koaxial mit dem verlängerten Körper (2) ist und von circa 10 mm lang ist.

7. Ein Stopfen nach Anspruch 1, umfassend eine Fensterung, die ein Loch durch das Gewinde ist, das Loch habende einen Durchmesser von 1 mm.

8. Ein Stopfen nach einem der vorhergehenden Ansprüche, mit einer Länge von 1,25 cm bis 3,5 cm.

9. Ein Stopfen nach einem der vorhergehenden Ansprüche, mit Durchmesser von 2mm bis 5 mm.

## Revendications

1. Une bonde (1) utilisée pour occlure un tube de Fallope par hystéroscopie, ladite bonde comprenant :
un corps allongé (2) ;
ledit corps allongé possédant un filetage externe (3) pour visser la bonde à l'intérieur et le long d'un canal intérieur,
ladite bonde comprenant une extrémité arrière comprenant une tête d'entrainement (13), ladite tête d'entrainement étant adaptée à être temporairement engagée par une portion d'un outil d'entrainement en cours d'utilisation, pour rotation de ladite bonde à l'intérieur et le long dudit canal intérieur,
ladite tête d'entrainement étant adaptée à être facilement détachable vis-à-vis dudit outil d'entrainement suite à l'achèvement de l'insertion de ladite bonde à l'intérieur dudit tube de Fallope ; et
**caractérisée en ce que**
ledit corps (2) et/ou ledit filetage externe (3) possède/possèdent une surface externe rugueuse et/ou piquée (4) pour réduire la migration postopératoire de ladite bonde ; et
ledit filetage (3) est fenêtré.

2. Une bonde (1) selon la revendication 1, dans laquelle une extrémité avant (6) du corps allongé (2) est fournie avec une pointe d'extension (7) possédant une surface externe lisse (8) et un embout arrondi (9).

3. Une bonde selon la revendication 1 ou la revendication 2, qui est moulée par injection à partir d'un matériau plastique synthétique rigide inerte et de classe biocompatible.

4. Une bonde selon la revendication 3, dans laquelle le matériau plastique est le polyéthylène.

5. Une bonde selon l'une des revendications précédentes, dans laquelle ladite rugosité et/ou ledit piquage possède jusqu'à 1 millimètre de profondeur.

6. Une bonde selon la revendication 2, dans laquelle la pointe d'extension (7) est coaxiale avec le corps allongé (2) et possède une longueur d'environ 10 millimètres.

7. Une bonde selon la revendication 1, comprenant un fenêtrage consistant d'une ouverture à travers ledit filetage, ladite ouverture possédant un diamètre de 1 millimètre.

8. Une bonde selon l'une des revendications précédentes, ayant une longueur de 1,25 centimètre à 3,5 centimètres.

9. Une bonde selon l'une des revendications précédentes, ayant un diamètre de 2 millimètres à 5 millimètres.
